# EUROPEAN PATENT SPECIFICATION

(11) **EP 3 939 984 B1**
(45) Date of publication and mention of the grant of the patent: **11.12.2024**
(21) Application number: 19920547.7
(22) Date of filing: 18.04.2019
(51) Int. Cl.: C07H 19/06, A61K 31/7068, A61K 31/7076, A61P 31/18

(54) **CRYSTAL FORM A OF 2'-FLUORO-4'-SUBSTITUTED NUCLEOSIDE ANALOG I AND PREPARATION METHOD THEREFOR AND USE THEREOF**
KRISTALLFORM EINES 2'-FLUOR-4'-SUBSTITUIERTEN NUCLEOSIDANALOGONS I UND VERFAHREN ZU SEINER HERSTELLUNG UND VERWENDUNG DAVON
FORME CRISTALLINE A DE L'ANALOGUE I DE NUCLÉOSIDE 2'-FLUORO-4'-SUBSTITUÉ, SON PROCÉDÉ DE PRÉPARATION ET SON UTILISATION

(30) Priority: 15.03.2019 CN 201910197742
(43) Date of publication of application: 19.01.2022
(73) Proprietor: Henan Genuine Biotech Co., Ltd., Henan 467036 (CN)
(72) Inventor: CHANG, Junbiao, Zhengzhou, Henan 450002 (CN); DU, Jinfa, Zhengzhou, Henan 450002 (CN); YI, Dongxu, Zhengzhou, Henan 450002 (CN)
(74) Representative: Scholl, Matthias
(86) International application number: PCT/CN2019/083217
(87) International publication number: WO 2020/186588

(56) References cited:
- WO-A1-2009/067409
- CN-A- 101 177 442
- CN-A- 102 000 103
- CN-A- 103 709 220
- CN-A- 108 503 681
- AALTONEN J ET AL: "Solid form screening - A review", EUROPEAN JOURNAL OF PHARMACEUTICS AND BIOPHARMACEUTICS, ELSEVIER SCIENCE PUBLISHERS B.V., AMSTERDAM, NL, vol. 71, no. 1, 1 January 2009 (2009-01-01), pages 23 - 37, XP025782069, ISSN: 0939-6411, [retrieved on 20080731], DOI: 10.1016/J.EJPB.2008.07.014
- MINO R CAIRA ED - MONTCHAMP JEAN-LUC: "Crystalline Polymorphism of Organic Compounds", TOPICS IN CURRENT CHEMISTRY; [TOPICS IN CURRENT CHEMISTRY], SPRINGER, BERLIN, DE, vol. 198, 1 January 1998 (1998-01-01), pages 163 - 208, XP008166276, ISSN: 0340-1022, [retrieved on 19990226], DOI: 10.1007/3-540-69178-2_5
- JOHN F BAUER: "Pharmaceutical Solids— The Amorphous Phase", JOURNAL OF VALIDATION TECHNOLOGY, vol. 15, no. 3, 1 January 2009 (2009-01-01), Netherlands, pages 63 - 68, XP055280414
- QIANG WANG ET AL: "Synthesis of new 2-deoxy-2-fluoro-4-azido nucleoside analogues as potent anti-HIV agents", EUROPEAN JOURNAL OF MEDICINAL CHEMISTRY, ELSEVIER, AMSTERDAM, NL, vol. 46, no. 9, 16 June 2011 (2011-06-16), pages 4178 - 4183, XP028278316, ISSN: 0223-5234, [retrieved on 20110623], DOI: 10.1016/J.EJMECH.2011.06.020
- DAVID B. SMITH ET AL: "The Design, Synthesis, and Antiviral Activity of Monofluoro and Difluoro Analogues of 4'-Azidocytidine against Hepatitis C Virus Replication: The Discovery of 4'-Azido-2'-deoxy-2'-fluorocytidine and 4'-Azido-2'-dideoxy-2',2'-difluorocytidine", JOURNAL OF MEDICINAL CHEMISTRY, vol. 52, no. 9, 14 May 2009 (2009-05-14), pages 2971 - 2978, XP055054142, ISSN: 0022-2623, DOI: 10.1021/jm801595c
- QIANG WANG ET AL: "FNC, a novel nucleoside analogue inhibits cell proliferation and tumor growth in a variety of human cancer cells", BIOCHEMICAL PHARMACOLOGY, ELSEVIER, US, vol. 81, no. 7, 4 January 2011 (2011-01-04), pages 848 - 855, XP028166488, ISSN: 0006-2952, [retrieved on 20110108], DOI: 10.1016/J.BCP.2011.01.001
- YU L ED - KWON ICK CHAN ET AL: "AMORPHOUS PHARMACEUTICAL SOLIDS: PREPARATION, CHARACTERIZATION AND STABILIZATION", ADVANCED DRUG DELIVERY REVIEWS, ELSEVIER, AMSTERDAM , NL, vol. 48, no. 1, 16 May 2001 (2001-05-16), pages 27 - 42, XP009065056, ISSN: 0169-409X, DOI: 10.1016/S0169-409X(01)00098-9
- WANG, QIANG ET AL.: "FNC, a Novel Nucleoside Analogue Inhibits Cell Proliferation and Tumor Growth in a Variety of Human Cancer Cells", BIOCHEMICAL PHARMACOLOGY, vol. 81, 8 January 2011 (2011-01-08), XP028166488, DOI: 20191203145518X

## Description

The disclosure relates to 2'-fluoro-4'-substituted nucleoside analogue I, and more particularly to a crystal form A of 2'-fluoro-4'-substituted nucleoside analogue I, and preparation method and application thereof, which belongs to the field of pharmaceutical chemistry.

Some nucleoside analogues exhibit significant antiviral activity, particularly, to fight against human immunodeficiency virus (HIV), hepatitis B virus (HBV) and hepatitis C virus (HCV), and thus are clinically used for the treatment of viral infections. In addition to the antiviral activity, some nucleosides also have anticancer activity. However, these drugs have certain defects. On the one hand, the efficacy of the drugs is limited, on the other hand, long-term use thereof poses serious toxicity and side effects and will produce drug resistance. Therefore, the design and synthesis of new nucleoside analogues is an important research direction for the discovery of new antiviral drugs. To find more effective nucleoside antiviral drugs, nucleosides have been modified in a variety of ways, among which fluorine-containing nucleoside analogues are an important category (Ismaili, H.M.A., WO 2001060315 A2). Qiang Wang et al. ("Synthesis of new 2'-deoxy-2'-fluoro-4'-azido nucleoside analogues as potent anti-HIV agents," European Journal of Medicinal Chemistry, vol. 46, no. 9, 2011, pp. 4178-4183) disclose 2'-deoxy-2'-beta-fluoro-4'-azidocytidine ("FNC"), a white solid. CN 103 709 220 B discloses a stereoisomer of FNC. David B. Smith et al. ("The design, synthesis, and antiviral activity of monofluoro and difluoro analogues of 4'-azidocytidine against hepatitis C virus replication: the discovery of 4'-azido-2'-deoxy-2'-fluorocytidine and 4'-azido-2'-dideoxy-2',2'-difluorocytidine," Journal of Medicinal Chemistry, vol. 52, no. 9, 2009, pp. 2971-2978) discloses FNC, an off-white solid.

The granted patent CN 100532388C of the inventor discloses a series of 2'-fluoro-4'-substituted nucleoside analogues, which have good antiviral activity. It is well-known that the solubility and bioavailability of a drug are subject to the crystal forms thereof. In addition, the stability, fluidity and compressibility thereof may also be different for different crystal forms. These physical and chemical properties have a certain impact on the application of the drug, thus affecting the therapeutic effect of the drug. In particular, the anti HIV activity of 2'-fluoro-4'-substituted nucleoside analogue I is high, and the clinical dosage for adults is only 1-3 mg. To ensure the quality of tablets and clinical medication effect, the crystal form with stable performance and easy to mix with excipients should be adopted. Therefore, studying the crystal form of 2'-fluoro-4'-substituted nucleoside analogue I is conducive to its use in drug processing and pharmaceutical composition. The crystal form of 2'-fluoro-4'-substituted nucleoside analogue I has not yet been reported.

One objective of the disclosure is to provide a crystal form A of 2'-fluoro-4'-substituted nucleoside analogue I. Another objective of the disclosure is to provide a preparation method of the crystal form A and an application of the crystal form A for use as an antiviral drug, particularly for an anti-AIDS drug. Still another objective of the disclosure is to provide an application of the crystal form A for use in the treatment of a cancer, particularly in the treatment of lung cancer, gastric cancer, colorectal cancer or lymphatic cancer, non-Hodgkin's lymphoma and leukemia.

To achieve above objectives, the following technical solutions are adopted.

2'-fluoro-4'-substituted nucleoside analogue I of the disclosure is represented by the following formula:

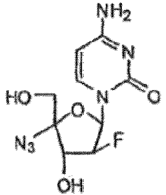

The crystal forms of the compound were systematically screened by means of unitary method, binary method, cooling, dissolution (positive and negative), diffusion and so on, and two crystal forms were found: crystal form A and amorphous crystal form B. The stabilities of the crystal form A and the crystal form B under high temperature, high humidity and light were evaluated respectively. The evaluation results showed that crystal form B could be gradually transformed into crystal form A within ten days under high humidity and high temperature conditions, and the crystal form A was stable under the same conditions. These experimental conditions were as follows: the high temperature condition is 60°C for five days and ten days; the high humidity condition is 40°C and 75% humidity for five and ten days; the light conditions were 4500 lux for five and ten days.

The compound I is dissolved in methanol, ethanol, isopropanol, ethyl acetate, dichloromethane or other solvents, and then the solvent is removed by a rotary evaporator to obtain a sheet amorphous solid B. The sheet amorphous solid B has obvious static electricity and is easily adsorbed on the container wall. After 10 days of storage at 40°C/75% humidity, 80-90% of the solid compound I with amorphous form B is gradually transformed into stable crystal form A. The compound of crystal form A is a loose solid. Because the dosage of the compound I for adults with AIDS is only 1-3 mg per day, which is a low amount, the uniform mixing of the compound with an excipient is crucial to ensure the anti HIV effect of the drug. The compound I in the form of sheet amorphous solid B is not easy to mix evenly with excipients (see Table 1), which adversely affects the controllability of drug efficacy. The crystal form A of the compound I is more stable than the amorphous crystal form B, and is easy to mix evenly with excipients, which is conducive to drug quality control and drug efficacy.

**Table 1 Content of compound I with amorphous B and crystal form A in tablets**

| Compound | Crystal form | Static | Drug content for each tablet* | Number of tablets arbitrarily selected |
|---|---|---|---|---|
| Crystal form of compound I | Amorphous B | Yes | 0.5 - 1.5 mg | 10 |
| | Crystal form | No | 0.95 - 1.05 mg | 10 |

| | | | | |
|---|---|---|---|---|
| *Each tablet contains an average of 1 mg of the compound I and 149 mg of excipients. The total weight of each tablet is 150 mg. | | | | |

The results of single crystal study show that the crystal form A of the compound I is monoclinic system and the space group is p2₁. The single crystal structure is as follows:

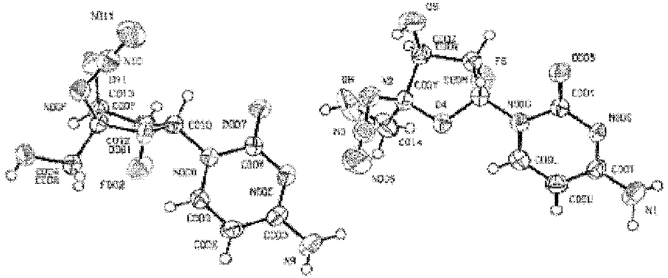

**Table 2 Characterization data of single crystal A of compound I**

| Identification of compound | Compound I |
|---|---|
| Empirical formula | C9 H11 F N6 O4 |
| Formula weight | 286.24 |
| Temperature | 303(2) K |
| Wavelength | 1.54178 Å |
| Crystal system | Monoclinic |
| Space group | P21 |
| Unit cell dimensions | a = 11.0901(10) Å α = 90°. |
| | b = 9.3505(8) Å β = 109.608(4)°. |
| | c = 12.0032(10) Å γ = 90°. |
| Volume | 1172.53(18) Å3 |
| Z | 4 |
| Density (calculated) | 1.621 Mg/m³ |

Further studies showed the compound I had significant inhibitory effects on many types of cancer, and its anticancer activity and anti-lymphatic cancer effect were verified in animal models. Experiments showed that the compound I had significant inhibitory effects on B-cell non-Hodgkin's lymphoma, lung cancer and leukemia, especially on non-Hodgkin's lymphoma.

The following advantages are associated with the crystal form A of 2'-fluoro-4'-substituted nucleoside analogue I of the disclosure. The 2'-fluoro-4'-substituted nucleoside analogues I have special structures including fluorine-containing groups and azide, which solves the shortcomings of toxicity and side effects and low activity of conventional D- and L-nucleoside analogues, and when applied to the preparation of anti HBV or anti HCV or anti HIV drugs and anti-tumor drugs, especially drugs fighting against lymphatic cancer and non-Hodgkin's lymphatic cancer, and exhibit good application value. The crystal form A of the compound has good stability, no static electricity, and is easy to mix evenly with excipients, which is conducive to the preparation of pharmaceutical preparations. The crystal form A of the compound I is mixed with excipients to significantly improve its uniformity in the tablets, which is conducive to drug quality control, reducing the generation of drug resistance, and ensuring the efficacy. Therefore, the crystal form A of the compound I of the disclosure can be used for preparing antiviral drugs, especially for preparing anti AIDS drugs. The crystal form A of the compound I can also be used for preparing anti-cancer drugs, especially for preparing drugs for fighting against B-cell non-Hodgkin's lymphoma, lung cancer, leukemia and so on.

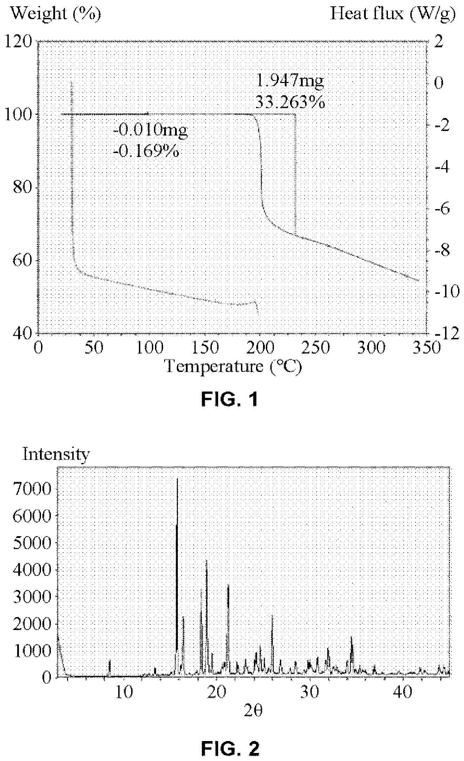

FIG. 1 is a differential scanning calorimetry (DSC)-thermogravimetric analysis (TGA) spectrum of a crystal form A of a compound I of the disclosure;
FIG. 2 is a CuKα-X-ray powder diffraction (XRPD) spectrum of a crystal form A of a compound I of the disclosure;

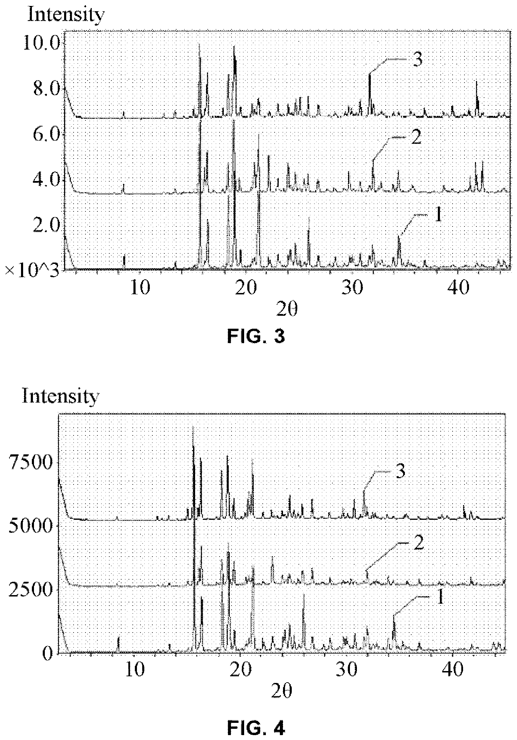

FIG. 3 is a stability comparison of CuKα-XRPD spectra of a crystal form A of a compound I of the disclosure under high temperature: line 1 shows the CuKα-XRPD spectrum of the crystal form A of the compound I; line 2 shows the CuKα-XRPD spectrum of the crystal form A of the compound I at 60°C for five days; and line 3 shows the CuKα-XRPD spectrum of the crystal form A of the compound I at 60°C for ten days;
FIG. 4 is a stability comparison of CuKα-XRPD spectra of a crystal form A of a compound I of the disclosure under high humidity: line 1 shows the CuKα-XRPD spectrum of the crystal form A of the compound I; line 2 shows the CuKα-XRPD spectrum of the crystal form A of the compound I at 40°C and 75% humidity for ten days; and line 3 shows the CuKα-XRPD spectrum of the crystal form A of the compound I at 40°C and 75% humidity for five days;

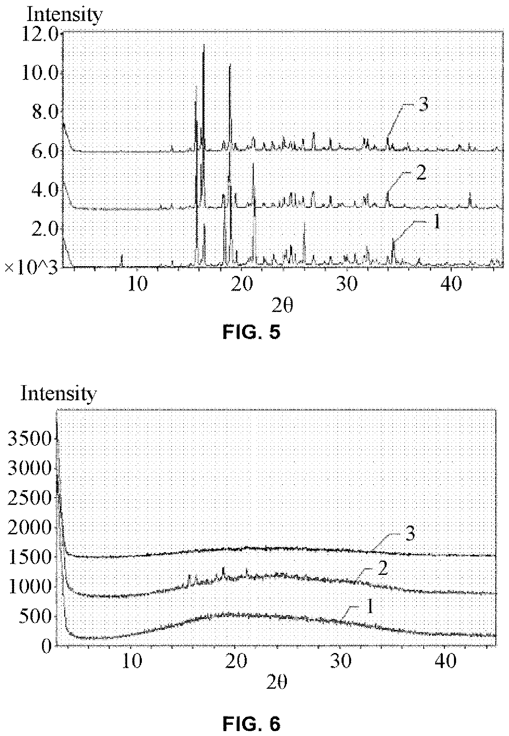

FIG. 5 is a stability comparison of CuKα-XRPD spectra of a crystal form A of a compound I of the disclosure under light condition: line 1 shows the CuKα-XRPD spectrum of the crystal form A of the compound I; line 2 shows the CuKα-XRPD spectrum of the crystal form A of the compound I at 4500 lux for ten days; and line 3 shows the CuKα-XRPD spectrum of the crystal form A of the compound I at 4500 lux for five days;
FIG. 6 is a stability comparison of CuKα-XRPD spectra of a crystal form B of a compound I of the disclosure under high temperature: line 1 shows the CuKα-XRPD spectrum of the crystal form B of the compound I; line 2 shows the CuKα-XRPD spectrum of the crystal form B of the compound I at 60°C for ten days; and line 3 shows the CuKα-XRPD spectrum of the crystal form B of the compound I at 60°C for five days;

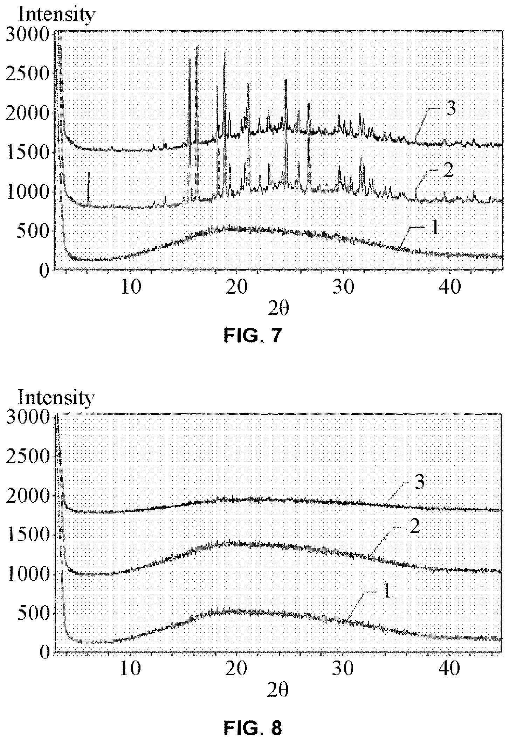

FIG. 7 is a stability comparison of CuKα-XRPD spectra of a crystal form B of a compound I of the disclosure under high humidity: line 1 shows the CuKα-XRPD spectrum of the crystal form B of the compound I; line 2 shows the CuKα-XRPD spectrum of the crystal form B of the compound I at 40°C and 75% humidity for ten days; and line 3 shows the CuKα-XRPD spectrum of the crystal form B of the compound I at 40°C and 75% humidity for five days;
FIG. 8 is a stability comparison of CuKα-XRPD spectra of a crystal form B of a compound I of the disclosure under light condition: line 1 shows the CuKα-XRPD spectrum of the crystal form B of the compound I; line 2 shows the CuKα-XRPD spectrum of the crystal form B of the compound I at 4500 lux for ten days; and line 3 shows the CuKα-XRPD spectrum of the crystal form B of the compound I at 4500 iux for five days; and

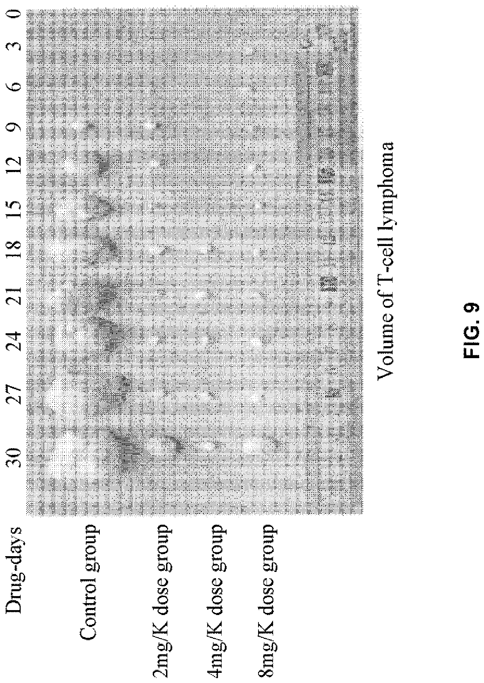

FIG. 9 is a picture showing anti-T-cell lymphoma activity of the crystal form A of the compound I of the disclosure in patient-derived tumor xenografts (PDX) mouse model.

To further illustrate, embodiments detailing a crystal form A of 2'-fluoro-4'-substituted nucleoside analogue I are described below. It should be noted that the following embodiments are intended to describe and not to limit the disclosure.

### Example 1 preparation of crystal form B

### 1.1 Suspension experiment

2'-fluoro-4'-substituted nucleoside analogue I of the disclosure was dissolved in one of the following solvents, and the solution concentration was 7-20 mg/MI. The solvent was removed using a rotatory evaporator, to yield a crystal form B.

The abovementioned solvent is: methanol, ethanol, n-propanol, isopropanol, N,N-dimethylformamide (DMF), ethyl acetate, isopropyl acetate, n-hexane, cyclohexane, water, ether, isopropyl ether, methyl tert butyl ether, 4-methyl-pentanone, tetrahydrofuran, acetonitrile, dichloromethane and chloroform.

### 1.2 Rapid evaporation experiment

100 mg of the compound I was added to excess methanol solution and heated up for dissolution. The mixed solution was placed in a vacuum rotatory evaporator at 50°C. After the solvent was removed, a white solid was obtained, which was in the form of crystal form B.

### Example 2 preparation of crystal form A

### 2.1 Recrystallization experiment

2'-fluoro-4'-substituted nucleoside analogue I of the disclosure was dissolved in methanol, ethanol, n-propanol or water and heated to reflux to prepare its saturated solution. Thereafter, the saturated solution was cooled and crystallized to obtain the crystal form A.

### 2.2 Liquid level diffusion experiment

The compound I was dissolved in a solvent at 60°C, and then an antisolvent was slowly added dropwise to the sample solution along the wall, cooled, and a solid was precipitated. The crystal form of the obtained solid is crystal form A.

The solvent was Methanol, DMF or water.

The antisolvent was N-hexane, cyclohexane, isopropyl ether or ethyl acetate.

### 2.3 Binary solvent experiment

15 mg of the compound I was added to a sample bottle, and then 3 mL of a binary mixed solvent was added. The solution was shaken in a 200-rpm shaker at 50°C for 48 h, and then filtered. The solution was allowed to slowly volatilize to remove the solvent, and the crystal form A was obtained.

Binary mixed solvent: methanol (n-propanol, ethyl acetate, n-hexane, cyclohexane, isopropyl ether, methyl tert-butyl ether (MTBE), acetone, acetonitrile, dichloromethane or toluene), DMF (n-propanol, ethyl acetate, n-hexane, cyclohexane, isopropyl ether, MTBE, acetone, acetonitrile, dichloromethane or toluene), water (n-propanol, ethyl acetate, n-hexane, cyclohexane, isopropyl ether, MTBE, acetone, acetonitrile, dichloromethane or toluene); the ratio of the binary mixed solvent: methanol: n-propanol = 1:4 (volume ratio), DMF: n-propanol = 1:4 (volume ratio), water: n-propanol = 1:4 (volume ratio), and so on.

The experimental results showed that the stable crystal form of the compound I was crystal form A. The crystal form A of the compound I had diffraction peaks at 2θ angles (±0.2): 8.56, 13.40, 15.76, 16.43, 18.38, 18.95, 19.49, 20.62, 20.86, 21.20, 25.99, 26.85, 27.89, 28.48, 29.78, 30.04, 30.84, 31.71, 31.96, 33.95, 34.47 through X-ray powder diffraction (XRPD) using CuKa radiation with a wavelength of λ=1.5418 Å (as shown in FIG. 2).

Diffraction peaks at 2θ angles and corresponding relative intensities (%) of the crystal form A of the compound I:

| 2θ (±0.2°) | Relative intensities I% |
|---|---|
| 8.56 | 8.6 |
| 13.40 | 4.2 |
| 15.76 | 100 |
| 16.43 | 29.5 |
| 18.38 | 42.7 |
| 18.95 | 58.1 |
| 19.49 | 10.7 |
| 20.62 | 4.9 |
| 20.86 | 6.2 |
| 21.20 | 45.8 |
| 25.99 | 29.4 |
| 26.85 | 7.0 |
| 27.89 | 2.5 |
| 28.48 | 6.2 |
| 29.78 | 6.0 |
| 30.04 | 6.8 |
| 30.84 | 8.0 |
| 31.71 | 6.7 |
| 31.96 | 12.9 |
| 33.95 | 6.2 |
| 34.47 | 18.7 |

### Conclusion:

1. The results of DSC and XRD showed that the crystal form A was the stable crystal form of the compound I.
2. The crystal form A exhibited good stability in stability evaluation.
3. The crystal form B was generally stable and can be transformed into crystal form A under appropriate conditions.
4. The crystal form B was transformed into crystal form A under high humidity and high temperature conditions for ten days.
5. When the crystal form A of the compound I was mixed with excipients, its uniformity in the tablets was significantly improved, which is conducive to the quality control of drugs, reducing the generation of drug resistance, and ensuring the efficacy.

### Example 3 Antiviral effect of crystal form A of compound I

The anti HIV activity of crystal form A of the compound I was determined according to the literature method (EUR. J. Med. Chem. 2011,464178).

### Example 4 Inhibitory effect of crystal form A of compound I

The anticancer (non-Hodgkin's lymphoma) activity of the crystal form A of the compound I was determined according to the literature method (Asian Pacific Journal of cancer prevention 2014, 15, 6829).

### Example 5 Anticancer effect of the crystal form A of the compound I on B-cell non-Hodgkin lymphoma, lung cancer, gastric cancer, colorectal cancer and leukemia

The anticancer activity of the crystal form A of the compound I on B-cell non-Hodgkin lymphoma, lung cancer, gastric cancer, colorectal cancer and leukemia was determined according to the literature method (Wang, Q. et al Biochemical Pharmacology 2011, 81, 848; Asian Pacific Journal of Cancer Prevention 2014, 15, 6829).

### Example 6 Anti-T-cell lymphoma activity of the crystal form A of the compound I in patient-derived tumor xenografts (PDX) mouse model

The inhibitory effect of the crystal form A of the compound I on T-cell lymphoma was determined by patient-derived tumor xenograft (PDX).

After the patient's T-cell lymphoid carcinoma tissue was sampled, the tissue was transferred to a specific pathogen free (SPF) animal room through a transfer window. In a super clean table, phosphate buffered saline (PBS) buffer with double antibodies, Dulbecco's modified eagle medium (DMEM) medium, Petri dish and ice bag were prepared. An appropriate amount of PBS and DMEM were poured to the Petri dishes on ice. The tissue was taken out and put into PBS for preliminary cleaning, and necrotic and normal tissue was removed. The tissue in PBS was transferred to DMEM. The remaining tissue was cut into small pieces of 20-30 mm³ patches, which were transplanted into the armpit and back of mice with puncture needles. Each mouse was inoculated with the tissue on one site. The tumors of the inoculated mice were taken out regularly, photographed, and the volume changes thereof were compared (see FIG. 9).

Through the experiments of patient-derived tumor xenografts mouse model, the crystal form A of the compound I could significantly inhibit the growth of T-cell lymphoma at the doses of 2 mg/kg, 4 mg/kg, and 8 mg/kg, respectively.

## Claims

1. A crystal form A of a compound I, 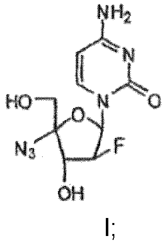
**characterized in that** the crystal form A is a monoclinic system and a space group thereof is p2₁, and a single crystal structure being as follows: 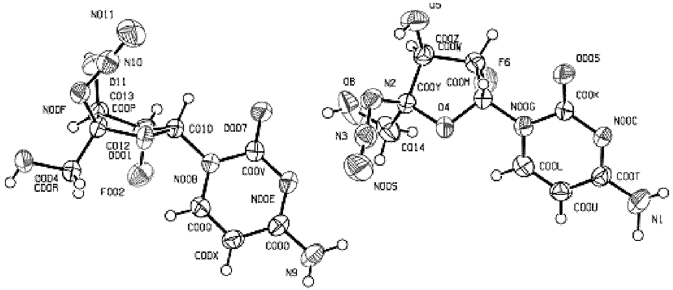
and a CuKα-X-ray powder diffraction (XRPD) spectrum of the crystal form A comprising diffraction peaks at diffraction angle 2θ positions of: 8.56, 13.40, 15.76, 16.43, 18.38, 18.95, 19.49, 20.62, 20.86, 21.20, 25.99, 26.85, 27.89, 28.48, 29.78, 30.04, 30.84, 31.71, 31.96, 33.95, 34.47, and an error range of 2θ is ±0.2 using CuKα radiation with a wavelength of λ=1.5418Å.

2. The crystal form of claim 1, wherein the CuKα-X-ray powder diffraction (XRPD) spectrum is shown in FIG. 2.

3. The crystal form A of claim 1 or 2 for use as an antiviral drug

4. The crystal form A for use of claim 3, wherein the crystal form A is an anti-AIDS drug.

5. The crystal form A of claim 1 or 2 for use in the treatment of a cancer

6. The crystal form A for use of claim 5, wherein the cancer is lung cancer, gastric cancer, colorectal cancer, leukemia, or lymphatic cancer.

7. The crystal form A for use of claim 6, wherein the cancer is B-cell non-Hodgkin's lymphoma.

## Patentansprüche

1. Kristallform A einer Verbindung I, 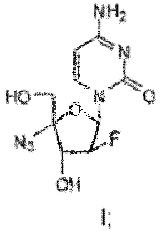
**dadurch gekennzeichnet, dass** die Kristallform A ein monoklines System ist und eine Raumgruppe davon p2₁ ist und die Einkristallstruktur wie folgt ist: 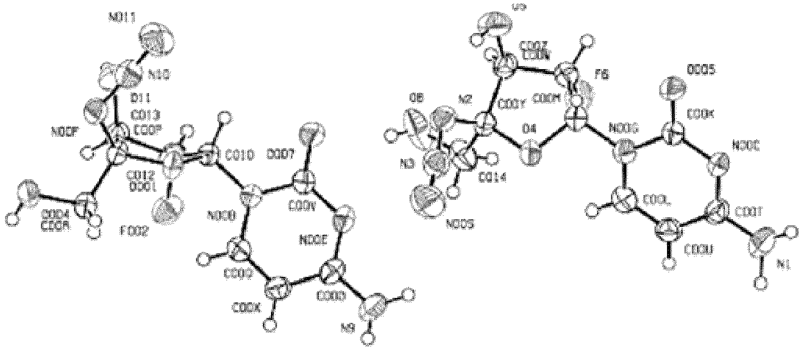
und ein CuKα-Röntgenpulverbeugungsspektrum (XRPD) der Kristall form A, umfassend Beugungsspitzen an Beugungswinkel 2θ-Positionen von: 8,56, 13,40, 15,76, 16,43, 18,38, 18,95, 19,49, 20,62, 20,86, 21,20, 25,99, 26,85, 27,89, 28,48, 29,78, 30,04, 30,84, 31,71, 31,96, 33,95, 34,47 und bei Verwendung von CuKα-Strahlung mit einer Wellenlänge von λ=1,5418 Å ein Fehlerbereich von 2θ ±0,2 beträgt.

2. Kristallform nach Anspruch 1, wobei das CuKα-Röntgenpulverbeugungsspektrum (XRPD) in FIG. 2 dargestellt ist.

3. Kristallform A nach Anspruch 1 oder 2 zur Verwendung als antivirales Arzneimittel.

4. Kristallform A zur Verwendung nach Anspruch 3, wobei die Kristallform A ein Anti-AIDS-Arzneimittel ist.

5. Kristallform A nach Anspruch 1 oder 2 zur Verwendung bei der Behandlung von Krebs.

6. Kristallform A zur Verwendung nach Anspruch 5, wobei die Krebserkrankung Lungenkrebs, Magenkrebs, Dickdarmkrebs, Leukämie oder Lymphdrüsenkrebs ist.

7. Kristallform A zur Verwendung nach Anspruch 6, wobei die Krebserkrankung ein B-Zell-Non-Hodgkin-Lymphom ist.

## Revendications

1. Forme cristalline A d'un composé I, 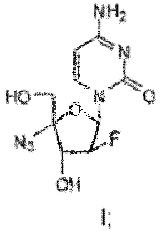
**caractérisée en ce que** la forme cristalline A est un système monoclinique et un groupe spatial de celui-ci est p2i, et une structure monocristalline étant comme suit : 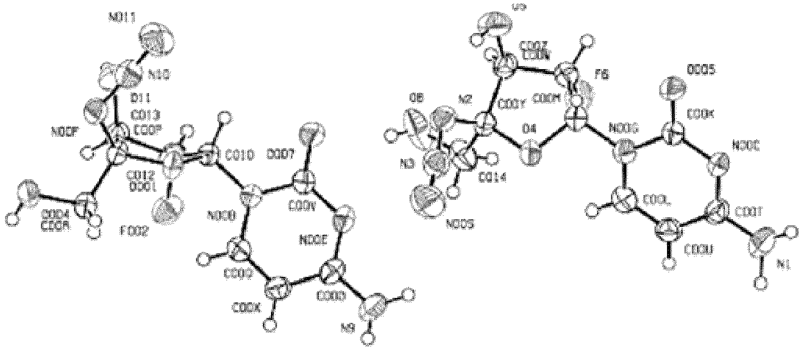
et un spectre de diffraction sur poudre des rayons X (XRPD) de rayonnement CuKα de la forme cristalline A comprenant des pics de diffraction au niveau des positions d'angle 2θ de diffraction : 8,56, 13,40, 15,76, 16,43, 18,38, 18,95, 19,49, 20,62, 20,86, 21,20, 25,99, 26,85, 27,89, 28,48, 29,78, 30,04, 30,84, 31,71, 31,96, 33,95, 34,47, et une plage d'erreur de 2θ est ±0,2 en utilisant un rayonnement CuKα avec une longueur d'onde de λ = 1,5418 Å.

2. Forme cristalline selon la revendication 1, ledit spectre de diffraction sur poudre des rayons X (XRPD) de rayonnement CuKα étant représenté à la figure 2.

3. Forme cristalline A selon la revendication 1 ou 2 destiné à être utilisé comme médicament antiviral.

4. Forme cristalline A destinée à être utilisée selon la revendication 3, ladite forme cristalline A étant un médicament anti-SIDA.

5. Forme cristalline A selon la revendication 1 ou 2 destiné à être utilisé dans le traitement d'un cancer.

6. Forme cristalline A destinée à être utilisée selon la revendication 5, ledit cancer étant le cancer du poumon, le cancer de l'estomac, le cancer colorectal, la leucémie ou le cancer lymphatique.

7. Forme cristalline A destinée à être utilisée selon la revendication 6, ledit cancer étant un lymphome non hodgkinien à lymphocytes B.
